(19) Europäisches Patentamt
European Patent Office
Office européen des brevets

(11) Veröffentlichungsnummer: **0 210 445 B1**

(12) **EUROPÄISCHE PATENTSCHRIFT**

(45) Veröffentlichungstag der Patentschrift: **09.05.90**

(51) Int. Cl.⁵: **A01N 35/06**, A01N 37/42

(21) Anmeldenummer: **86108826.8**

(22) Anmeldetag: **28.06.86**

(54) **Mittel zur Regulierung des Pflanzenwachstums.**

(30) Priorität: **04.07.85 DE 3523862**

(43) Veröffentlichungstag der Anmeldung: **04.02.87 Patentblatt 87/6**

(45) Bekanntmachung des Hinweises auf die Patenterteilung: **09.05.90 Patentblatt 90/19**

(84) Benannte Vertragsstaaten: **BE CH DE FR GB IT LI NL**

(56) Entgegenhaltungen: **EP-A- 0 123 001**

(73) Patentinhaber: **BASF Aktiengesellschaft, Carl-Bosch-Strasse 38, D-6700 Ludwigshafen(DE)**

(72) Erfinder: **Jahn, Dieter, Dr., Burgunder Weg 8, D-6803 Edingen-Neckarhausen(DE)**
Erfinder: **Keil, Michael, Dr., Fontanestrasse 4, D-6713 Freinsheim(DE)**
Erfinder: **Kolassa, Dieter, Dr., Moltkestrasse 8, D-6700 Ludwigshafen(DE)**
Erfinder: **Schirmer, Ulrich, Dr., Berghalde 79, D-6900 Heidelberg(DE)**
Erfinder: **Becker, Rainer, Dr., Im Haseneck 22, D-6702 Bad Duerkheim(DE)**
Erfinder: **Jung, Johann, Prof. Dr., Hardenburgstrasse 19, D-6703 Limburgerhof(DE)**
Erfinder: **Rademacher, Wilhelm, Dr., Austrasse 1, D-6703 Limburgerhof(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft die Verwendung von Cyclohexenonderivaten zur Regulierung des Wachstums von Pflanzen sowie ein Verfahren zur Regulierung des Pflanzenwachstums.

Es ist bekannt, daß bestimmte 2-Acyl-3-hydroxy-cyclohex-2-en-1-one in das Pflanzenwachstum regulierend eingreifen (EP-A 123 001 und EP-A 126 713). Hingewiesen wird auch auf die EP-A 156 773.

Es wurde nun gefunden, daß sich Cyclohexenonderivate der Formel I

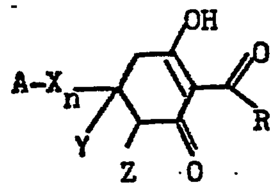

(I),

in der

A Alkoxycarbonyl mit 2 bis 5 Kohlenstoffatomen, Carboxyl, Cyano oder Trifluormethyl,

X einen unverzweigten oder verzweigten Alkylenrest mit 1 bis 7 Kohlenstoffatomen oder einen Cycloalkylenrest mit 3 bis 7 Kohlenstoffatomen,

n 0 oder 1 mit der Maßgabe, daß n nicht 0 ist, wenn A für Alkoxycarbonyl oder Carboxyl steht,

Y Wasserstoff oder Methyl,

Z Wasserstoff, Alkoxycarbonyl mit 2 bis 5 Kohlenstoffatomen oder Cyano und

R Alkyl und Alkoxyalkyl mit jeweils bis zu 4 Kohlenstoffatomen, Cyclopropyl, Benzyl, Phenylethyl oder Acyloxyalkyl mit bis zu 6 Kohlenstoffatomen bedeuten, oder eines seiner landwirtschaftlich annehmbaren Salze,

hervorragend zur Regulierung des Pflanzenwachstums verwenden lassen.

In Formel I bedeutet A beispielsweise Methoxycarbonyl, Ethoxycarbonyl, Propoxycarbonyl, Butoxycarbonyl, Carboxyl, Cyano oder Trifluormethyl,

X steht z.B. für Methylen, Ethylen, Trimethylen, Tetramethylen, Methylethylen, Dimethylpentamethylen, Ethyltrimethylen, 1,2-, 1,3- oder 1,4-Cyclohexylen oder 1,2-Cyclopropylen,

Z beispielsweise für Methoxycarbonyl, Ethoxycarbonyl, Cyano oder Wasserstoff, und R z.B. für Methyl, Ethyl, n-Propyl, i-Propyl, n-Butyl, sec-Butyl, i-Butyl, tert-Butyl, Cyclopropyl, Benzyl, Phenylethyl, Methoxyethyl, Acetoxymethyl oder Propionoxyethyl.

Als Salze der Verbindungen der Formel I kommen landwirtschaftlich brauchbare Salze, beispielsweise Alkalimetallsalze, insbesondere Kalium- oder Natriumsalze, Erdalkalimetallsalze insbesondere Calcium-, Magnesium- oder Bariumsalze, Mangan-, Kupfer-, Zink- oder Eisensalze sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Aus der DE-OS 3 440 410 ist bekannt, daß die genannten Cyclohexenonderivate der Formel I als Zwischenprodukte für die Synthese von Herbiziden dienen; dort ist auch ihre Herstellung beschrieben.

Verschiedene Syntheseverfahren für Cyclohexenonderivate sind auch den oben genannten europäischen Patentanmeldungen zu entnehmen.

Beispielhaft sei die Synthese von 2-Butyryl-3-hydroxy-4-methoxycarbonyl-5-(4-methoxycarbonylbutyl)-cyclohex-2-en-1-on beschrieben.

### Herstellungsbeispiel

81 Gew.-Teile 3-Butyryloxy-4-methoxycarbonyl-5-(4-methoxycarbonylbutyl)-cyclohex-2-en-1-on und 8 Gew.-Teile 4-Dimethylaminopyridin wurden in wasserfreiem Dichlormethan gelöst und 48 Stunden bei Raumtemperatur gerührt. Man wusch mit 1O gew.%iger Salzsäure und Wasser, trocknete über Natriumsulfat und destillierte das Lösungsmittel unter vermindertem Druck ab. Man erhielt 2-Butyryl-3-hydroxy-4-methoxy-carbonyl-5-(4-methoxycarbonylbutyl)cyclohex-2-en-1-on als Öl (Verbindung Nr. 4) $^1$H-NMR: δ x O,92 (t), 2,95 (t), 3,65 (s).

Die in der folgenden Tabelle aufgeführten Verbindungen wurden auf entsprechende Weise hergestellt, soweit sie mit physikalischen Daten versehen sind. Die übrigen in der Tabelle aufgeführten Verbindungen können nach Wahl entsprechender Ausgangsstoffe auf die gleiche Weise erhalten werden und lassen eine ähnliche biologische Wirkung erwarten wie die untersuchten Verbindungen. Bei asymmetrischen Alkylenresten X soll dabei deren in der Tabelle links stehende Bindung an den Substituenten A gebunden sein.

| Verbindung Nr. | A | X | n | Y | Z | R | Physik. Daten* |
|---|---|---|---|---|---|---|---|
| 1 | $CF_3$ | 1,4-Cyclohexylen | 1 | H | H | n-Propyl | 0,98 (t), 3,0 (t) |
| 2 | $CF_3$ | 1,4-Cyclohexylen | 1 | H | H | Ethyl | |
| 3 | $COOCH_3$ | $-(CH_2)_4-$ | 1 | H | $COOCH_3$ | Butyl | 0,92 (t), 3,69 (s) 4,81 (t) |
| 4 | $COOCH_3$ | $-(CH_2)_4-$ | 1 | H | $COOCH_3$ | n-Propyl | 0,92 (t), 2,95 (t), 3,65 (s) |
| 5 | $COOCH_3$ | $-(CH_2)_4-$ | 1 | H | $COOCH_3$ | Ethyl | 1,17 (t), 2,35 (t), 3,83 (s) |
| 6 | $COOCH_3$ | $-(CH_2)_4-$ | 1 | H | $COOCH_3$ | Methyl | 2,59 (s), 3,68 (s), 3,78 (s) |
| 7 | $COOCH_3$ | $-CH(CH_3)CH_2)-$ | 1 | H | $COOCH_3$ | Ethyl | 1,13 (t), 3,05 (q), 3,7 (s) |
| 8 | $COOCH_3$ | $-CH_2CH(CH_3)-$ | 1 | H | $COOCH_3$ | Methyl | |
| 9 | $COOCH_3$ | $-CH(CH_3)CH_2-$ | 1 | H | $COOCH_3$ | n-Propyl | 0,93 (t), 3,7 (s), 3,8 (s) |
| 10 | $COOC_2H_5$ | $-CH_2CH(CH_3)-$ | 1 | H | $COOCH_3$ | n-Propyl | |
| 11 | $COOn-C_4H_9$ | $-CH_2CH(CH_3)-$ | 1 | H | $COOCH_3$ | n-Propyl | |
| 12 | CN | $-CH_2CH(CH_3)-$ | 1 | H | $COOCH_3$ | n-Propyl | |
| 13 | $CF_3$ | $-CH(CH_3)CH_2-$ | 1 | H | $COOCH_3$ | n-Propyl | |
| 14 | CN | $-CH(CH_3)CH_2-$ | 1 | H | $COOCH_3$ | Ethyl | |
| 15 | $COOC_2H_5$ | 1,2-Cyclopropylen | 1 | H | $COOCH_3$ | n-Propyl | |
| 16 | $COOC_2H_5$ | 1,2-Cyclopropylen | 1 | H | $COOCH_3$ | Ethyl | |
| 17 | $CF_3$ | | 0 | $CH_3$ | H | n-Propyl | 1,05 (t), 1,35 (s), 3,05 (t) |
| 18 | $CF_3$ | | 0 | $CH_3$ | H | Ethyl | |

* Als physikalische Daten werden Schmelzpunkt [°C], Brechungsindex sowie $^1$H-NMR Signale, (ppm) aufgeführt.
Dabei bedeuten: s + Singulett, d + Dublett, t + Triplett, q + Quartett, m + Multiplett

| Verbindung Nr. | A | X | n | Y | Z | R | Physik. Daten* |
|---|---|---|---|---|---|---|---|
| 19 | $CF_3$ | | 0 | $CH_3$ | $COOCH_3$ | n-Propyl | 1,4 (s), 1,65 (q), 3,8 (s) |
| 20 | $CF_3$ | | 0 | $CH_3$ | $COOCH_3$ | Ethyl | |
| 21 | COOH | | 0 | H | $COOCH_3$ | Ethyl | |
| 22 | COOH | | 0 | H | $COOCH_3$ | n-Propyl | |
| 23 | $-COOCH_3$ | $-CH_2CH(CH_3)(CH_2)_3-$ | 1 | H | $COOCH_3$ | n-Propyl | 0,9 (t), 3,6 (s), 3,7 (s) |
| 24 | $-COOCH_3$ | $-CH_2CH(CH_3)(CH_2)_3-$ | 1 | H | $COOCH_3$ | Ethyl | |
| 25 | $-COOCH_3$ | 1,2-Cyclopropylen | 1 | H | H | Methyl | |
| 26 | $-COOCH_3$ | 1,2-Cyclopropylen | 1 | H | H | Ethyl | |
| 27 | $-COOCH_3$ | 1,2-Cyclopropylen | 1 | H | H | n-Propyl | |
| 28 | $-COO-nC_4H_9$ | 1,2-Cyclopropylen | 1 | H | H | Methyl | |
| 29 | $-COO-n-C_4H_9$ | 1,2-Cyclopropylen | 1 | H | H | Ethyl | |
| 30 | $-COO-n-C_4H_9$ | 1,2-Cyclopropylen | 1 | H | H | n-Propyl | |
| 31 | $-COO-tert-C_4H_9$ | 1,2-Cyclopropylen | 1 | H | H | Methyl | |
| 32 | $-COO-tert-C_4H_9$ | 1,2-Cyclopropylen | 1 | H | H | Ethyl | |
| 33 | $-COO-tert-C_4H_9$ | 1,2-Cyclopropylen | 1 | H | H | n-Propyl | |
| 34 | -COOH | 1,2-Cyclopropylen | 1 | H | H | Methyl | |
| 35 | -COOH | 1,2-Cyclopropylen | 1 | H | H | Ethyl | |
| 36 | -COOH | 1,2-Cyclopropylen | 1 | H | H | n-Propyl | 1,0 (t), 3,0 (t) |
| 37 | -CN | 1,2-Cyclopropylen | 1 | H | H | Methyl | |
| 38 | -CN | 1,2-Cyclopropylen | 1 | H | H | Ethyl | |
| 39 | -CN | 1,2-Cyclopropylen | 1 | H | H | n-Propyl | |
| 40 | $-COOCH_3$ | 1,2-Cyclohexylen | 1 | H | H | Methyl | |
| 41 | $-COOCH_3$ | 1,2-Cyclohexylen | 1 | H | H | Ethyl | |
| 42 | $-COOCH_3$ | 1,2-Cyclohexylen | 1 | H | H | n-Propyl | |
| 43 | $-COO-n-C_4H_9$ | 1,2-Cyclohexylen | 1 | H | H | Methyl | |
| 44 | $-COO-n-C_4H_9$ | 1,2-Cyclohexylen | 1 | H | H | Ethyl | |

| Verbindung Nr. | A | X | n | Y | Z | R | Physik. Daten* |
|---|---|---|---|---|---|---|---|
| 45 | $-COO-n-C_4H_9$ | 1,2-Cyclohexylen | 1 | H | H | n-Propyl | |
| 46 | $-COO-tert-C_4H_9$ | 1,2-Cyclohexylen | 1 | H | H | Methyl | |
| 47 | $-COO-tert-C_4H_9$ | 1,2-Cyclohexylen | 1 | H | H | Ethyl | |
| 48 | $-COO-tert-C_4H_9$ | 1,2-Cyclohexylen | 1 | H | H | n-Propyl | |
| 49 | -COOH | 1,2-Cyclohexylen | 1 | H | H | Methyl | |
| 50 | -COOH | 1,2-Cyclohexylen | 1 | H | H | Ethyl | |
| 51 | -COOH | 1,2-Cyclohexylen | 1 | H | H | n-Propyl | |
| 52 | -CN | 1,2-Cyclohexylen | 1 | H | H | Methyl | |
| 53 | -CN | 1,2-Cyclohexylen | 1 | H | H | Ethyl | |
| 54 | -CN | 1,2-Cyclohexylen | 1 | H | H | n-Propyl | |
| 55 | $-COOCH_3$ | 1,3-Cyclohexylen | 1 | H | H | Methyl | |
| 56 | $-COOCH_3$ | 1,3-Cyclohexylen | 1 | H | H | Ethyl | |
| 57 | $-COOCH_3$ | 1,3-Cyclohexylen | 1 | H | H | n-Propyl | |
| 58 | $-COO-n-C_4H_9$ | 1,3-Cyclohexylen | 1 | H | H | Methyl | |
| 59 | $-COO-n-C_4H_9$ | 1,3-Cyclohexylen | 1 | H | H | Ethyl | |
| 60 | $-COO-n-C_4H_9$ | 1,3-Cyclohexylen | 1 | H | H | n-Propyl | |
| 61 | $-COO-tert-C_4H_9$ | 1,3-Cyclohexylen | 1 | H | H | Methyl | |
| 62 | $-COO-tert-C_4H_9$ | 1,3-Cyclohexylen | 1 | H | H | Ethyl | |
| 63 | $-COO-tert-C_4H_9$ | 1,3-Cyclohexylen | 1 | H | H | n-Propyl | |
| 64 | -COOH | 1,3-Cyclohexylen | 1 | H | H | Methyl | |
| 65 | -COOH | 1,3-Cyclohexylen | 1 | H | H | Ethyl | |
| 66 | -COOH | 1,3-Cyclohexylen | 1 | H | H | n-Propyl | |
| 67 | -CN | 1,3-Cyclohexylen | 1 | H | H | Methyl | |
| 68 | -CN | 1,3-Cyclohexylen | 1 | H | H | Ethyl | |
| 69 | -CN | 1,3-Cyclohexylen | 1 | H | H | n-Propyl | |

| Verbindung Nr. | A | X | n | Y | Z | R | Physik. Daten* |
|---|---|---|---|---|---|---|---|
| 70 | $-COOCH_3$ | 1,4-Cyclohexylen | 1 | H | H | Methyl | |
| 71 | $-COOCH_3$ | 1,4-Cyclohexylen | 1 | H | H | Ethyl | |
| 72 | $-COOCH_3$ | 1,4-Cyclohexylen | 1 | H | H | n-Propyl | |
| 73 | $-COO-n-C_4H_9$ | 1,4-Cyclohexylen | 1 | H | H | Methyl | |
| 74 | $-COO-n-C_4H_9$ | 1,4-Cyclohexylen | 1 | H | H | Ethyl | |
| 75 | $-COO-n-C_4H_9$ | 1,4-Cyclohexylen | 1 | H | H | n-Propyl | |
| 76 | $-COO-tert-C_4H_9$ | 1,4-Cyclohexylen | 1 | H | H | Methyl | |
| 77 | $-COO-tert-C_4H_9$ | 1,4-Cyclohexylen | 1 | H | H | Ethyl | |
| 78 | $-COO-tert-C_4H_9$ | 1,4-Cyclohexylen | 1 | H | H | n-Propyl | |
| 79 | -COOH | 1,4-Cyclohexylen | 1 | H | H | Methyl | |
| 80 | -COOH | 1,4-Cyclohexylen | 1 | H | H | Ethyl | |
| 81 | -COOH | 1,4-Cyclohexylen | 1 | H | H | n-Propyl | |
| 82 | -CN | 1,4-Cyclohexylen | 1 | H | H | Methyl | |
| 83 | -CN | 1,4-Cyclohexylen | 1 | H | H | Ethyl | |
| 84 | -CN | 1,4-Cyclohexylen | 1 | H | H | n-Propyl | |
| 85 | $-COOCH_3$ | Methylen | 1 | H | H | Methyl | |
| 86 | $-COOCH_3$ | Methylen | 1 | H | H | Ethyl | |
| 87 | $-COOCH_3$ | Methylen | 1 | H | H | n-Propyl | |
| 88 | $-COO-n-C_4H_9$ | Methylen | 1 | H | H | Methyl | |
| 89 | $-COO-n-C_4H_9$ | Methylen | 1 | H | H | Ethyl | |
| 90 | $-COO-n-C_4H_9$ | Methylen | 1 | H | H | n-Propyl | |
| 91 | $-COO-tert-C_4H_9$ | Methylen | 1 | H | H | Methyl | |
| 92 | $-COO-tert-C_4H_9$ | Methylen | 1 | H | H | Ethyl | |

| Verbindung Nr. | A | X | n | Y | Z | R | Physik. Daten* |
|---|---|---|---|---|---|---|---|
| 93 | $-COO-tert-C_4H_9$ | Methylen | 1 | H | H | n-Propyl | |
| 94 | $-COOH$ | Methylen | 1 | H | H | Methyl | |
| 95 | $-COOH$ | Methylen | 1 | H | H | Ethyl | |
| 96 | $-COOH$ | Methylen | 1 | H | H | n-Propyl | |
| 97 | $-CN$ | Methylen | 1 | H | H | Methyl | |
| 98 | $-CN$ | Methylen | 1 | H | H | Ethyl | |
| 99 | $-CN$ | Methylen | 1 | H | H | n-Propyl | |
| 100 | $-COOCH_3$ | Ethylen | 1 | H | H | Methyl | |
| 101 | $-COOCH_3$ | Ethylen | 1 | H | H | Ethyl | |
| 102 | $-COOCH_3$ | Ethylen | 1 | H | H | n-Propyl | |
| 103 | $-COO-n-C_4H_9$ | Ethylen | 1 | H | H | Methyl | |
| 104 | $-COO-n-C_4H_9$ | Ethylen | 1 | H | H | Ethyl | |
| 105 | $-COO-n-C_4H_9$ | Ethylen | 1 | H | H | n-Propyl | |
| 106 | $-COO-tert-C_4H_9$ | Ethylen | 1 | H | H | Methyl | |
| 107 | $-COO-tert-C_4H_9$ | Ethylen | 1 | H | H | Ethyl | |
| 108 | $-COO-tert-C_4H_9$ | Ethylen | 1 | H | H | n-Propyl | |
| 109 | $-COOH$ | Ethylen | 1 | H | H | Methyl | |
| 110 | $-COOH$ | Ethylen | 1 | H | H | Ethyl | |
| 111 | $-COOH$ | Ethylen | 1 | H | H | n-Propyl | |
| 112 | $-CN$ | Ethylen | 1 | H | H | Methyl | |
| 113 | $-CN$ | Ethylen | 1 | H | H | Ethyl | |
| 114 | $-CN$ | Ethylen | 1 | H | H | n-Propyl | |
| 115 | $-COOCH_3$ | Trimethylen | 1 | H | H | Methyl | |
| 116 | $-COOCH_3$ | Trimethylen | 1 | H | H | Ethylen | |

| Verbindung Nr. | A | X | n | Y | Z | R | Physik. Daten |
|---|---|---|---|---|---|---|---|
| 117 | $-COOCH_3$ | Trimethylen | 1 | H | H | n-Propyl | 0,95 (t), 3,01 (t) 3,69 (s) |
| 118 | $-COO-n-C_4H_9$ | Trimethylen | 1 | H | H | Methyl | |
| 119 | $-COO-n-C_4H_9$ | Trimethylen | 1 | H | H | Ethyl | |
| 120 | $-COO-n-C_4H_9$ | Trimethylen | 1 | H | H | n-Propyl | |
| 121 | $-COO-tert-C_4H_9$ | Trimethylen | 1 | H | H | Methyl | |
| 122 | $-COO-tert-C_4H_9$ | Trimethylen | 1 | H | H | Ethyl | |
| 123 | $-COO-tert-C_4H_9$ | Trimethylen | 1 | H | H | n-Propyl | |
| 124 | -COOH | Trimethylen | 1 | H | H | Methyl | |
| 125 | -COOH | Trimethylen | 1 | H | H | Ethyl | |
| 126 | -COOH | Trimethylen | 1 | H | H | n-Propyl | 0,89 (t), 2,93 (t), 12,0 (s) |
| 127 | -CN | Trimethylen | 1 | H | H | Methyl | |
| 128 | -CN | Trimethylen | 1 | H | H | Ethyl | |
| 129 | -CN | Trimethylen | 1 | H | H | n-Propyl | |
| 130 | $-COOCH_3$ | Tetramethylen | 1 | H | H | Methyl | 1,42 (m), 2,63 (s), 3,70 (s) |
| 131 | $-COOCH_3$ | Tetramethylen | 1 | H | H | Ethyl | 1,13 (t), 3,05 (q), 3,66 (s) |
| 132 | $-COOCH_3$ | Tetramethylen | 1 | H | H | n-Propyl | 1,02 (t), 3,00 (t), 3,69 (s) |
| 133 | $-COO-n-C_4H_9$ | Tetramethylen | 1 | H | H | Methyl | 0,93 (t), 2,61 (s), 4,07 (t) |
| 134 | $-COO-n-C_4H_9$ | Tetramethylen | 1 | H | H | Ethyl | 0,93 (t), 3,05 (q), 4,07 (t) |
| 135 | $-COO-n-C_4H_9$ | Tetramethylen | 1 | H | H | n-Propyl | 2,32 (t), 3,02 (t), 4,10 (t) |
| 136 | $-COO-tert-C_4H_9$ | Tetramethylen | 1 | H | H | Methyl | |
| 137 | $-COO-tert-C_4H_9$ | Tetramethylen | 1 | H | H | Ethyl | |

| Verbindung Nr. | A | X | n | Y | Z | R | Physik. Daten |
|---|---|---|---|---|---|---|---|
| 138 | $-COO-tert-C_4H_9$ | Tetramethylen | 1 | H | H | n-Propyl | |
| 139 | $-COOH$ | Tetramethylen | 1 | H | H | Methyl | 1,40 (m), 2,30 (t), 2,59 (s) |
| 140 | $-COOH$ | Tetramethylen | 1 | H | H | Ethyl | 97 - 98 |
| 141 | $-COOH$ | Tetramethylen | 1 | H | H | n-Propyl | 64 |
| 142 | $-CN$ | Tetramethylen | 1 | H | H | Methyl | |
| 143 | $-CN$ | Tetramethylen | 1 | H | H | Ethyl | |
| 144 | $-CN$ | Tetramethylen | 1 | H | H | n-Propyl | |
| 145 | $-COOCH_3$ | $-CH(CH_3)(CH_2)_3-$ | 1 | H | H | Methyl | |
| 146 | $-COOCH_3$ | $-CH(CH_3)(CH_2)_3-$ | 1 | H | H | Ethyl | |
| 147 | $-COOCH_3$ | $-CH(CH_3)(CH_2)_3-$ | 1 | H | H | n-Propyl | |
| 148 | $-COO-n-C_4H_9$ | $-CH(CH_3)(CH_2)_3-$ | 1 | H | H | Methyl | |
| 149 | $-COO-n-C_4H_9$ | $-CH(CH_3)(CH_2)_3-$ | 1 | H | H | Ethyl | |
| 150 | $-COO-n-C_4H_9$ | $-CH(CH_3)(CH_2)_3-$ | 1 | H | H | n-Propyl | |
| 151 | $-COO-tert-C_4H_9$ | $-CH(CH_3)(CH_2)_3-$ | 1 | H | H | Methyl | |
| 152 | $-COO-tert-C_4H_9$ | $-CH(CH_3)(CH_2)_3-$ | 1 | H | H | Ethyl | |
| 153 | $-COO-tert-C_4H_9$ | $-CH(CH_3)(CH_2)_3-$ | 1 | H | H | n-Propyl | |
| 154 | $-COOH$ | $-CH(CH_3)(CH_2)_3-$ | 1 | H | H | Methyl | |
| 155 | $-COOH$ | $-CH(CH_3)(CH_2)_3-$ | 1 | H | H | Ethyl | |
| 156 | $-COOH$ | $-CH(CH_3)(CH_2)_3-$ | 1 | H | H | n-Propyl | |
| 157 | $-CN$ | $-CH(CH_3)(CH_2)_3-$ | 1 | H | H | Methyl | |
| 158 | $-CN$ | $-CH(CH_3)(CH_2)_3-$ | 1 | H | H | Ethyl | |
| 159 | $-CN$ | $-CH(CH_3)(CH_2)_3-$ | 1 | H | H | n-Propyl | |
| 160 | $-COOCH_3$ | $-CH(CH_3)CH_2-$ | 1 | H | H | Methyl | |
| 161 | $-COOCH_3$ | $-CH(CH_3)CH_2-$ | 1 | H | H | Ethyl | |
| 162 | $-COOCH_3$ | $-CH(CH_3)CH_2-$ | 1 | H | H | n-Propyl | |

| Verbindung Nr. | A | X | n | Y | Z | R | Physik. Daten |
|---|---|---|---|---|---|---|---|
| 163 | $-COO-n-C_4H_9$ | $-CH(CH_3)CH_2-$ | 1 | H | H | Methyl | |
| 164 | $-COO-n-C_4H_9$ | $-CH(CH_3)CH_2-$ | 1 | H | H | Ethyl | |
| 165 | $-COO-n-C_4H_9$ | $-CH(CH_3)CH_2-$ | 1 | H | H | n-Propyl | |
| 166 | $-COO-tert-C_4H_9$ | $-CH(CH_3)CH_2-$ | 1 | H | H | Methyl | |
| 167 | $-COO-tert-C_4H_9$ | $-CH(CH_3)CH_2-$ | 1 | H | H | Ethyl | |
| 168 | $-COO-tert-C_4H_9$ | $-CH(CH_3)CH_2-$ | 1 | H | H | n-Propyl | |
| 169 | -COOH | $-CH(CH_3)CH_2-$ | 1 | H | H | Methyl | |
| 170 | -COOH | $-CH(CH_3)CH_2-$ | 1 | H | H | Ethyl | 1,3 (m), 2,5 (m), 3,1 (q) |
| 171 | -COOH | $-CH(CH_3)CH_2-$ | 1 | H | H | n-Propyl | 1,0 (t), 3,0 (q) |
| 172 | -CN | $-CH(CH_3)CH_2-$ | 1 | H | H | Methyl | |
| 173 | -CN | $-CH(CH_3)CH_2-$ | 1 | H | H | Ethyl | |
| 174 | -CN | $-CH(CH_3)CH_2-$ | 1 | H | H | n-Propyl | |
| 175 | $-COOCH_3$ | $-CH_2CH(CH_3)-$ | 1 | H | H | Methyl | |
| 176 | $-COOCH_3$ | $-CH_2CH(CH_3)-$ | 1 | H | H | Ethyl | |
| 177 | $-COOCH_3$ | $-CH_2CH(CH_3)-$ | 1 | H | H | n-Propyl | |
| 178 | $-COO-n-C_4H_9$ | $-CH_2CH(CH_3)-$ | 1 | H | H | Methyl | |
| 179 | $-COO-n-C_4H_9$ | $-CH_2CH(CH_3)-$ | 1 | H | H | Ethyl | |
| 180 | $-COO-n-C_4H_9$ | $-CH_2CH(CH_3)-$ | 1 | H | H | n-Propyl | |
| 181 | $-COO-tert-C_4H_9$ | $-CH_2CH(CH_3)-$ | 1 | H | H | Methyl | |
| 182 | $-COO-tert-C_4H_9$ | $-CH_2CH(CH_3)-$ | 1 | H | H | Ethyl | |
| 183 | $-COO-tert-C_4H_9$ | $-CH_2CH(CH_3)-$ | 1 | H | H | n-Propyl | |
| 184 | -COOH | $-CH_2CH(CH_3)-$ | 1 | H | H | Methyl | |
| 185 | -COOH | $-CH_2CH(CH_3)-$ | 1 | H | H | Ethyl | |
| 186 | -COOH | $-CH_2CH(CH_3)-$ | 1 | H | H | n-Propyl | |

| Verbindung Nr. | A | X | n | Y | Z | R | Physik. Daten |
|---|---|---|---|---|---|---|---|
| 187 | -CN | $-CH_2CH(CH_3-)-$ | 1 | H | H | Methyl | |
| 188 | -CN | $-CH_2CH(CH_3)-$ | 1 | H | H | Ethyl | |
| 189 | -CN | $-CH_2CH(CH_3)-$ | 1 | H | H | n-Propyl | |
| 190 | $-COOCH_3$ | $-(CH_2)_3CH(CH_3)CH_2-$ | 1 | H | H | Methyl | |
| 191 | $-COOCH_3$ | $-(CH_2)_3CH(CH_3)CH_2-$ | 1 | H | H | Ethyl | |
| 192 | $-COOCH_3$ | $-(CH_2)_3CH(CH_3)CH_2-$ | 1 | H | H | n-Propyl | 1,0 (t), 3,1 (t), 3,7 (s) |
| 193 | $-COO-n-C_4H_9$ | $-(CH_2)_3CH(CH_3)CH_2-$ | 1 | H | H | Methyl | |
| 194 | $-COO-n-C_4H_9$ | $-(CH_2)_3CH(CH_3)CH_2-$ | 1 | H | H | Ethyl | |
| 195 | $-COO-n-C_4H_9$ | $-(CH_2)_3CH(CH_3)CH_2-$ | 1 | H | H | n-Propyl | |
| 196 | $-COO-tert-C_4H_9$ | $-(CH_2)_3CH(CH_3)CH_2-$ | 1 | H | H | Methyl | |
| 197 | $-COO-tert-C_4H_9$ | $-(CH_2)_3CH(CH_3)CH_2-$ | 1 | H | H | Ethyl | |
| 198 | $-COO-tert-C_4H_9$ | $-(CH_2)_3CH(CH_3)CH_2-$ | 1 | H | H | n-Propyl | |
| 199 | -COOH | $-(CH_2)_3CH(CH_3)CH_2-$ | 1 | H | H | Methyl | |
| 200 | -COOH | $-(CH_2)_3CH(CH_3)CH_2-$ | 1 | H | H | Ethyl | |
| 201 | -COOH | $-(CH_2)_3CH(CH_3)CH_2-$ | 1 | H | H | n-Propyl | |
| 202 | -CN | $-(CH_2)_3CH(CH_3)CH_2-$ | 1 | H | H | Methyl | |
| 203 | -CN | $-(CH_2)_3CH(CH_3)CH_2$ | 1 | H | H | Ethyl | |
| 204 | -CN | $-(CH_2)_3CH(CH_3)CH_2$ | 1 | H | H | n-Propyl | |
| 205 | $-COOCH_3$ | $-CH(CH_3)(CH_2)_2CH(CH_3)CH_2-$ | 1 | H | H | Methyl | |
| 206 | $-COOCH_3$ | $-CH(CH_3)(CH_2)_2CH(CH_3)CH_2-$ | 1 | H | H | Ethyl | |
| 207 | $-COOCH_3$ | $-CH(CH_3)(CH_2)_2CH(CH_3)CH_2-$ | 1 | H | H | n-Propyl | |
| 208 | $-COO-n-C_4H_9$ | $-CH(CH_3)(CH_2)_2CH(CH_3)CH_2-$ | 1 | H | H | Methyl | |
| 209 | $-COO-n-C_4H_9$ | $-CH(CH_3)(CH_2)_2CH(CH_3)CH_2-$ | 1 | H | H | Ethyl | |
| 210 | $-COO-n-C_4H_9$ | $-CH(CH_3)(CH_2)_2CH(CH_3)CH_2-$ | 1 | H | H | n-Propyl | |

| Verbindung Nr. | A | X | n | Y | Z | R | Physik. Daten |
|---|---|---|---|---|---|---|---|
| 211 | $-COO-tert-C_4H_9$ | $-CH(CH_3)(CH_2)_2CH(CH_3)CH_2-$ | 1 | H | H | Methyl | |
| 212 | $-COO-tert-C_4H_9$ | $-CH(CH_3)(CH_2)_2CH(CH_3)CH_2$ | 1 | H | H | Ethyl | |
| 213 | $-COO-tert-C_4H_9$ | $-CH(CH_3)(CH_2)_2CH(CH_3)CH_2$ | 1 | H | H | n-Propyl | |
| 214 | $-COOH$ | $-CH(CH_3)(CH_2)_2CH(CH_3)CH_2-$ | 1 | H | H | Methyl | |
| 215 | $-COOH$ | $-CH(CH_3)(CH_2)_2CH(CH_3)CH_2-$ | 1 | H | H | Ethyl | |
| 216 | $-COOH$ | $-CH(CH_3)(CH_2)_2CH(CH_3)CH_2-$ | 1 | H | H | n-Propyl | |
| 217 | $-CN$ | $-CH(CH_3)(CH_2)_2CH(CH_3)CH_2-$ | 1 | H | H | Methyl | |
| 218 | $-CN$ | $-CH(CH_3)(CH_2)_2CH(CH_3)CH_2-$ | 1 | H | H | Ethyl | |
| 219 | $-CN$ | $-CH(CH_3)(CH_2)_2CH(CH_3)CH_2-$ | 1 | H | H | n-Propyl | |
| 220 | $-COOCH_3$ | $-(CH_2)_2CH(C_2H_5)-$ | 1 | H | H | Methyl | |
| 221 | $-COOCH_3$ | $-(CH_2)_2CH(C_2H_5)-$ | 1 | H | H | Ethyl | 0,91 (t), 3,06 (q), 3,70 (s) |
| 222 | $-COOCH_3$ | $-(CH_2)_2CH(C_2H_5)-$ | 1 | H | H | n-Propyl | |
| 223 | $-COO-n-C_4H_9$ | $-(CH_2)_2CH(C_2H_5)-$ | 1 | H | H | Methyl | |
| 224 | $-COO-n-C_4H_9$ | $-(CH_2)_2CH(C_2H_5)-$ | 1 | H | H | Ethyl | |
| 225 | $-COO-n-C_4H_9$ | $-(CH_2)_2CH(C_2H_5)-$ | 1 | H | H | n-Propyl | |
| 226 | $-COO-tert-C_4H_9$ | $-(CH_2)_2CH(C_2H_5)-$ | 1 | H | H | Methyl | |
| 227 | $-COO-tert-C_4H_9$ | $-(CH_2)_2CH(C_2H_5)-$ | 1 | H | H | Ethyl | |
| 228 | $-COO-tert-C_4H_9$ | $-(CH_2)_2CH(C_2H_5)-$ | 1 | H | H | n-Propyl | |
| 229 | $-COOH$ | $-(CH_2)_2CH(C_2H_5)-$ | 1 | H | H | Methyl | |
| 230 | $-COOH$ | $-(CH_2)_2CH(C_2H_5)-$ | 1 | H | H | Ethyl | 0,9 (t), 1,1 (t), 3,1 (q) |
| 231 | $-COOH$ | $-(CH_2)_2CH(C_2H_5)-$ | 1 | H | H | n-Propyl | |
| 232 | $-CN$ | $-(CH_2)_2CH(C_2H_5)-$ | 1 | H | H | Methyl | |
| 233 | $-CN$ | $-(CH_2)_2CH(C_2H_5)-$ | 1 | H | H | Ethyl | |
| 234 | $-CN$ | $-(CH_2)_2CH(C_2H_5)-$ | 1 | H | H | n-Propyl | |
| 235 | $-COOH$ | $-CH(CH_3)(CH$ | | | | | |

| Verbindung Nr. | A | X | n | Y | Z | R | Physik. Daten |
|---|---|---|---|---|---|---|---|
| 236 | $-CF_3$ | 1,3-Cyclohexylen | 1 | H | H | n-Propyl | 0,99 (t), 3,02 (t) |
| 237 | -CN | $-CH(CH_3)-$ | 1 | H | H | n-Propyl | |
| 238 | COO-tert-$C_4H_9$ | $-CH(CH_3)-$ | 1 | H | H | n-Propyl | 0,95 (t), 1,4 (s), 3,0 (t) |
| 239 | $-COOCH_3$ | $-CH(CH_3)(CH_2)_2CH(CH_3)CH_2$ | 1 | H | H | n-Propyl | 1,4920 |
| 240 | -COOH | $-CH(CH_3)-$ | 1 | H | H | Ethyl | 2,97 (q), 12,35 (s) 17,90 (s) |
| 241 | -COO-n-$C_4H_9$ | $-CH(CH_3)-$ | 1 | H | H | Ethyl | 0,95 (t), 3,07 (q), 4,11 (t) |
| 242 | $-COOCH_3$ | $-CH(CH_3)-$ | 1 | H | H | Ethyl | 1,13 (t), 1,21 (d), 3,72 (s) |
| 243 | $-COOCH_3$ | $-CH(CH_3)-$ | 1 | H | $COOCH_3$ | Ethyl | 3,03 (m), 3,71 (s), 3,82 (s) |
| 244 | -COOH | $-C(CH_3)_2-$ | 1 | H | H | Propyl | 0,98 (t), 1,25 (s), 3,01 (t) |
| 245 | $-COOCH_3$ | $-C(CH_3)_2-$ | 1 | H | H | Propyl | 0,98 (t), 1,22 (s), 3,72 (s) |
| 246 | -COOH | $-(CH_2)_2CH(CH_3)-$ | 1 | H | H | Ethyl | 0,96 (d), 1,14 (t), 3,05 (q) |
| 247 | $-COOCH_3$ | $-(CH_2)_2CH(CH_3)-$ | 1 | H | H | Ethyl | 0,91 (d), 3,03 (q), 3,68 (s) |
| 248 | $-COOCH_3$ | $-(CH_2)_2CH(CH_3)-$ | 1 | H | H | Propyl | 0,95 (m), 3,00 (t), 3,70 (s) |
| 249 | $-COOCH_3$ | $-(CH_2)_2CH(CH_3)-$ | 1 | H | $COOCH_3$ | Propyl | 0,94 (m), 2,98 (t), 3,68 (s) |
| 250 | -COOH | $-(CH_2)_4-$ | 1 | H | H | Butyl | 0,92 (t), 2,37 (t), 3,02 (t) |

| Verbindung Nr. | A | X | n | Y | Z | R | Physik. Daten |
|---|---|---|---|---|---|---|---|
| 251 | $-COO-n-C_4H_9$ | $-(CH_2)_4-$ | 1 | H | H | Butyl | 0,95 (m), 3,01 (t), 4,09 (t) |
| 252 | $-COOCH_3$ | $-(CH_2)_4-$ | 1 | H | H | Butyl | 0,95 (t), 3,02 (t), 3,72 (s) |
| 253 | -COOH | $-(CH_2)_2CH(CH_3)CH_2-$ | 1 | H | H | Ethyl | 0,89 (d), 1,17 (t), 3,04 (q) |
| 254 | -COOH | $-(CH_2)_2CH(CH_3)CH_2-$ | 1 | H | H | Butyl | 0,92 (m), 2,32 (t), 3,01 (t) |
| 255 | $-COOCH_3$ | $-(CH_2)_2CH(CH_3)CH_2-$ | 1 | H | H | Ethyl | 0,90 (d), 3,05 (q), 3,67 (s) |
| 256 | $-COOCH_3$ | $-(CH_2)_2CH(CH_3)CH_2-$ | 1 | H | H | Butyl | 0,90 (m), 3,=2 (t), 3,68 (s) |
| 257 | $-COO-n-C_4H_9$ | $-(CH_2)_2CH(CH_3)CH_2-$ | 1 | H | H | Methyl | 0,90 (m), 2,62 (s), 4,07 (t) |
| 258 | -COOH | $-(CH_2)_2CH(CH_3)CH_2-$ | 1 | H | H | Methyl | 0,92 (d), 2,33 (t), 2,62 (s) |
| 259 | -COOH | $-(CH_2)_2CH(CH_3)CH_2-$ | 1 | H | H | Propyl | 0,91 (d), 2,33 (t), 3,02 (t) |
| 260 | $-COOCH_3$ | $-(CH_2)_2CH(CH_3)CH_2-$ | 1 | H | H | Methyl | 0,88 (d), 2,61 (s), 3,68 (s) |
| 261 | $-COOCH_3$ | $-(CH_2)_2CH(CH_3)CH_2-$ | 1 | H | H | Propyl | 0,89 (d), 3,01 (t), 3,69 (s) |
| 262 | $-COO-n-C_4H_9$ | $-(CH_2)_2CH(CH_3)CH_2-$ | 1 | H | H | Ethyl | 1,18 (t), 3,07 (q), 4,08 (t) |
| 263 | $-COO-n-C_4H_9$ | $-(CH_2)_2CH(CH_3)CH_2-$ | 1 | H | H | Butyl | 2,29 (t), 3,03 (t), 4,08 (t) |
| 264 | $-COOCH_3$ | $-(CH_2)_2CH(CH_3)CH_2-$ | 1 | H | $COOCH_3$ | Propyl | 0,90 (m), 3,70 (s), 3,79 (s) |

| Verbindung Nr. | A | X | n | Y | Z | R | Physik. Daten |
|---|---|---|---|---|---|---|---|
| 265 | $-COOCH_3$ | $-(CH_2)_2CH(CH_3)CH_2-$ | 1 | H | $COOCH_3$ | Methyl | 2,62 (s), 3,67 (s), 3,78 (s) |
| 266 | $-COOCH_3$ | $-(CH_2)_2CH(CH_3)CH_2-$ | 1 | H | $COOCH_3$ | Ethyl | 1,12 (t), 3,04 (q), 3,67 (s) |
| 267 | $-COOCH_3$ | $-(CH_2)_2CH(CH_3)CH_2-$ | 1 | H | $COOCH_3$ | Butyl | 0,92 (m), 3,68 (s), 3,79 (s) |
| 268 | $-COO-n-C_4H_9$ | $-(CH_2)_2CH(CH_3)CH_2-$ | 1 | H | H | Propyl | 2,28 (t), 3,01 (t), 4,07 (t) |

| Verbindung Nr. | Salz von Verbindung Nr. | Kation(en) |
|---|---|---|
| 269 | 141 | 2 $Na^+$ |
| 270 | 141 | $Ca^{2+}$ |
| 271 | 139 | 2 $Na^+$ |
| 269 | 141 | 2 $Na^+$ |
| 272 | 258 | $Ca^{2+}$ |
| 273 | 258 | 2 $Na^+$ |
| 274 | 140 | 2 $Na^+$ |
| 275 | 139 | $Ca^{2+}$ |
| 276 | 250 | 2 $Na^+$ |
| 277 | 253 | $Ca^{2+}$ |
| 278 | 253 | 2 $Na^+$ |
| 279 | 140 | 2 Benzyltrimethylammor |
| 280 | 258 | 2 Benzyltrimethylammon |
| 281 | 259 | 2 $Na^+$ |
| 282 | 254 | 2 $Na^+$ |
| 283 | 253 | 2 Benzyltrimethylammon |
| 284 | 139 | 2 Benzyltrimethylammor |

Die Cyclohexenonderivate der Formel I können praktisch alle Entwicklungsstadien einer Pflanze verschiedenartig beeinflussen und werden deshalb als Wachstumsregulatoren eingesetzt.

Die erfindungsgemäß zu verwendenden Wirkstoffe können den Kulturpflanzen sowohl vom Samen her (als Saatgutbeizmittel) als auch über den Boden, d.h. durch die Wurzel sowie – besonders bevorzugt – durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,01 bis 12 kg/ha bevorzugt 0,25 bis 3 kg/ha als ausreichend zu betrachten.

Die Cyclohexenonderivate I können in Form üblicher Formulierungen angewendet werden wie Lösungen, Emulsionen, Suspensionen, Stäube, Pulver, Pasten und Granulate.

Die Cyclohexenonderivate I können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit anderen Wachstumsregulatoren treten auch synergistische Effekte auf, d.h. die Wirksamkeit des Kombinationsproduktes kann größer sein als die addierten Wirksamkeiten der Einzelkomponenten.

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. In anderen Versuchen wurden wäßrige Aufbereitungen vor dem Auflaufen der Pflanzen auf das Substrat appliziert. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Beispielsweise zeigt die Verbindung Nr. 140 deutlich wachstumsretardierende Eigenschaften, u.a. bei Weizen, Gerste und Reis.

Gleichlaufend zur Reduzierung des Längenwachstums stieg die Farbintensität der Blätter an. Der erhöhte Chlorophyllgehalt läßt eine ebenfalls erhöhte Photosyntheserate und damit erhöhte Ertragsbildung erwarten.

Die bioregulatorischen Eigenschaften der Verbindungen I wurden in verschiedenen Testverfahren untersucht. Als Vergleichssubstanzen dienten dabei

"A" Chlormequatchlorid
"B" Beispiel Nr. 13 aus EP 0 123 001 A1
"C" Beispiel Nr. 15 aus EP 0 123 001 A1
"D" Daminozid
"E" Mepiquatchlorid

1. Tests mit Keimlingen unter Gewächshausbedingungen

Zur Bestimmung der wachstumsregulierenden Eigenschaften der Prüfsubstanzen wurden Testpflanzen auf ausreichend mit Nährstoffen versorgtem Kultursubstrat in Kunststoffgefäßen (ca. 12,5 cm Durchmesser; Volumen ca. 500 ml) angezogen.

Die Testsubstanzen wurden in wäßriger Aufbereitung entweder am Tage der Einsaat (Vorauflaufverfahren) oder nach erfolger Keimung (Nachauflaufverfahren) auf das Substrat gegossen.

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Versuch 1

Sommergerste, Sorte "Aramir"
Vorauflauf-Bodenbehandlung

In diesem Versuch wiesen die Verbindungen 130, 131, 134 und 140 einen deutlich stärkeren Einfluß auf die Wuchshöhe auf, als die Vergleichsverbindungen A, B und C', ohne daß es zu einer Schädigung der Sämlinge kam.

Versuch 2

Reis, "Nihonbare"
Nachauflauf-Bodenbehandlung

Hierbei erwies sich die Verbindung 140 in verschiedenen Anwendungskonzentrationen deutlich wirksamer als A oder C, wobei A (Cycocel) völlig unwirksam war.

2. Reduzierung der Halmlänge und Ertragsbildung bei Reis

Jeweils 10 Pflanzen der Reissorte "Girona" wurden in Kunststoffgefäßen nach Kich/Brauckmann unter Gewächshausbedingungen kultiviert. Pro Gefäß wurden 4,5 kg eines Torfsubstrates verwendet, das mit einer ausreichenden Menge an mineralischen Nährsalzen versorgt wurde. Das Substrat wurde mit Wasser überstaut.

Bei einer Wuchshöhe der Pflanzen von 21 cm wurden die zu testenden Verbindungen in wäßriger Aufbereitung auf das Substrat gegossen. Dies entspricht weitgehend dem im Reisanbau häufig praktizierten Verfahren eines soil drenching.

Hierbei bewirkte die Verbindung 140 eine stärkere Einkürzung der Reispflanzen als die Vergleichssubstanz "C". Es ist mithin zu erwarten, daß die Lagerneigung der Pflanzen unter dem Einfluß von Verbindung 140 geringer ist als unter der Einwirkung von "C". Weiterhin zeigt sich, daß zumindest bei einer Aufwandmenge von 5 mg Wirkstoff pro Gefäß ein deutlich verbesserter Ertrag und ein günstigerer harvest index (Verhältnis vom Kornertrag zu Strohmasse) mit der Verbindung 140 erzielt werden. Die Vergleichssubstanz "C" bewirkte dagegen einen verminderten Ertrag bei ungünstigerem harvest index.

3. Reiskeimlingstest

Junge Reiskeimlinge (Sorte "Girona") wurden in Nährlösungen kultiviert, die die jeweiligen Wirkstoffe in unterschiedlichen Konzentrationen enthielten. Nach sechs Tagen Kultur bei 25°C im Dauerlicht wurde die Wirkstoffkonzentration bestimmt, die das Längenwachstum der zweiten Blattscheide um 50% verminderte ($=KI_{50}$).

(Details bei: W. Rademacher und J. Jung, Z. Acker- und Pflanzenbau 150, 363–371 (1981)).

Aus den erhaltenen Daten geht hervor, daß die Cyclohexenonderivate I bei deutlich geringerer Aufwandmenge den gleichen Effekt erzielen wie die Vergleichssubstanzen A, D und E.

Die untersuchten Verbindungen waren im einzelnen die mit den Nummern 6, 130, 131, 132, 133, 134, 135, 140, 141, 251, 269, 271, 274, 275, 279, 280 und 284.

## Patentansprüche

Verwendung von Cyclohexenonderivaten der Formel I

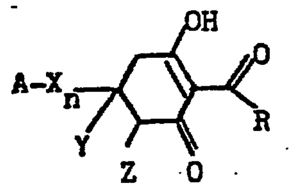

(I),

in der A Alkoxycarbonyl mit 2 bis 5 Kohlenstoffatomen, Carboxyl, Cyano oder Trifluormethyl,

X einen unverzweigten oder verzweigten Alkylenrest mit 1 bis 7 Kohlenstoffatomen oder einen Cycloalkylenrest mit 3 bis 7 Kohlenstoffatomen,

n 0 oder 1 mit der Maßgabe, daß n nicht 0 ist, wenn A für Alkoxycarbonyl oder Carboxyl steht,

Y Wasserstoff oder Methyl,

Z Wasserstoff, Alkoxycarbonyl mit 2 bis 5 Kohlenstoffatomen oder Cyano und

R Alkyl und Alkoxyalkyl mit jeweils bis zu 4 Kohlenstoffatomen, Cyclopropyl, Benzyl, Phenylethyl oder Acyloxyalkyl mit bis zu 6 Kohlenstoffatomen bedeuten, oder eines seiner landwirtschaftlich annehmbaren Salze

zur Regulierung des Pflanzenwachstums.

## Claims

Use of a cyclohexenone derivative of the formula I

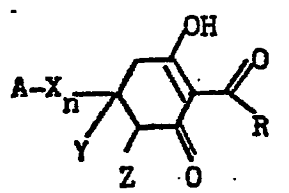

(I)

where

A is alkoxycarbonyl of 2 to 5 carbon atoms, carboxyl, cyano or trifluoromethyl,

X is straight-chain or branched alkylene of 1 to 7 carbon atoms or cycloalkylene of 3 to 7 carbon atoms,

n is 0 or 1, with the proviso that n is not 0 when A is alkoxycarbonyl or carboxyl,

Y is hydrogen or methyl,

Z is hydrogen, alkoxycarbonyl of 2 to 5 carbon atoms or cyano and

R is alkyl or alkoxyalkyl, each of not more than 4 carbon atoms, cyclopropyl, benzyl, phenylethyl or acyloxyalkyl of not more than 6 carbon atoms,

or one of its agriculturally acceptable salts, for regulating plant growth.

## Revendications

Utilisation pour la régulation de la croissance des plantes de dérivés de la cyclohexénone répondant à la formule I

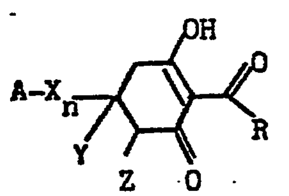

(I),

dans laquelle A représente un groupe alcoxycarbonyle en C2–C5, carboxyle, cyano ou trifluorométhyle,

X représente un groupe alkylène à chaîne droite ou ramifiée en C1–C7 ou un groupe cycloalkylène en C3–C7,

n est égal à 0 ou 1, sous réserve que n ne peut être égal à 0 lorsque A représente un groupe alcoxycarbonyle ou carboxyle,

Y représente l'hydrogène ou un groupe méthyle,

Z représente l'hydrogène, un groupe alcoxycarbonyle en C2–C5 ou cyano et

R représente un groupe alkyle ou alcoxyalkyle contenant chacun jusqu'à 4 atomes de carbone, cyclopropyle, benzyle, phényléthyle ou acyloxyalkyle contenant jusqu'à 6 atomes de carbone, ou de l'un de leurs sels acceptables pour les usages agricoles.